# EUROPEAN PATENT APPLICATION

(11) **EP 2 009 028 A1**
(43) Date of publication of application: **31.12.2008**
(21) Application number: 07301163.7
(22) Date of filing: 27.06.2007
(51) Int. Cl.: C07K 16/30, A61K 39/395, A61P 35/00, A61P 35/04

(54) **Combination of a conventional anti-cancer treatment with anti-CD44 antibody administration for treating solid tumors**

(71) Applicant: MONOCLONAL ANTIBODIES THERAPEUTICS, 91000 Evry (FR); INSTITUT NATIONAL DE LA SANTE ET DE LA RECHERCHE MEDICALE (INSERM), 75013 Paris (FR); CENTRE NATIONAL DE LA RECHERCHE SCIENTIFIQUE (CNRS), 75016 Paris (FR); Institut Curie, 75005 Paris (FR)
(72) Inventor: Smadja-Joffe, Florence, 92260 Fontenay aux Roses (FR); Boumsell, Laurence, 75015 Paris (FR); Poupon, Marie-France, 94260 Fresnes (FR); Marangoni, Elisabetta, 75014 Paris (FR); Kadouche, Jean, 75013 Paris (FR); Lecomte, Nicolas, 92160 Antony (FR)
(74) Representative: Warcoin, Jacques

(57) **Abstract**

The present invention concerns the use of an anti-CD44 antibody or an antigen binding fragment thereof for the manufacture of a drug for treating a solid tumor, preventing solid tumor rclapsc, or preventing solid tumor metastasis in a patient, wherein said patient is simultaneously treated or has been previously treated by another anti-cancer treatment. The other anti-cancer treatment is usually a conventional anti-cancer treatment and may be selected from the group consisting of chemotherapy, surgery, radiotherapy, hormonotherapy, and/or immunotherapy. The anti-CD44 antibody preferably recognizes a CD44 epitope in the same region of the CD44 molecule than the P245 antibody. The invention is notably suitable for breast cancer.

## Description

The present invention concerns the use of an anti-CD44 antibody or an antigen binding fragment thereof for the manufacture of a drug for treating a solid tumor, preventing solid tumor relapse, or preventing solid tumor metastasis in a patient, wherein said patient is simultaneously treated or has been previously treated by another anti-cancer treatment. The other anti-cancer treatment is usually a conventional anti-cancer treatment and may be selected from the group consisting of chemotherapy, surgery, radiotherapy, hormonotherapy, and/or immunotherapy. The anti-CD44 antibody preferably recognizes a CD44 epitope in the same region of the CD44 molecule than the P245 antibody. The invention is notably suitable for breast cancer.

Breast cancer is the most common cancer of women. Despite recent progresses in therapy, which have improved survival, mortality remains too high due to escape to treatment with local recurrences and the development of distant metastases. It is therefore essential to develop new targeted therapies to reduce recurrences and consequently breast cancer mortality.

Recently, rare populations of tumor cells designated as "tumor initiating cells" or "T-IC" have been identified with the ability to drive tumor formation and supporting the cancer stem cell hypothesis. This hypothesis postulates that tumors arise from stem cells that drive tumorigenesis, giving rise to a large population of progenitors that make up the bulk of the tumor but would lack tumorigenic potential.

Properties of cancer stem cells mean that they are able to give rise to the original cancer by regenerating the tumor cells. They have been shown to be tumorigenic (1-5) and resistant to radiotherapy *in vitro* (6). They are supposed to be resistant to treatments and therefore responsible of relapse after apparent tumor response *in vivo.* In cases in which tumor is eradicated by chemotherapy but is followed by a relapse, a possible reason is that the cancer stem cells are drug-resistant and have not been killed. Therapeutic approaches that specifically target cancer stem cells should prevent or reduce the risk of relapse and metastasis.

Cancer stem cells have been identified first in acute and chronic leukemia (7-8), then in solid tumors such as breast cancers (1), brain tumors (5), prostate cancer (9) and more recently in colon (4,2) and head and neck cancers (10). They have been isolated using stem cell markers, some of which have been shown to be shared by different tissues, like the adhesion molecule CD44, detected in breast, prostate and head and neck cancer stem cells, and which is also expressed by normal healthy mammary tissue stem cells (11).

The discovery of cancer stem cells markers has made their isolation possible and created a new appealing field of therapeutic possibilities. However, the mere identification of phenotypic markers is clearly not enough and target molecules, which might be used for therapeutic purposes, have yet to be identified.

The research team which first identified a population of tumor-initiating cells in breast cancer characterized by a CD44⁺CD24^{-/low} phenotype, has proposed several target molecules which might be useful for treating breast cancer, and especially breast cancer relapse, by an inhibitory effect on the population of CD44⁺CD24^{-/low} tumor-initiating cells.

For instance, in WO 03/050502 application (12), this research team identifies Notch 4 and its ligands Delta 1, Delta 2, Delta-like ligand 4(D114), Jagged 1 and Jagged 2, as well as Maniac fringe as potential therapeutic targets for treating breast cancer by targeting the CD44+CD24-/low population of tumor-initiating cells.

In WO 2006/138275 (13) also, this research team identifies further potential therapeutic targets for treating breast cancer by targeting the CD44⁺CD24-^{/low} population of tumor-initiating cells, such as c-Met, EDG2 or DCBLD2.

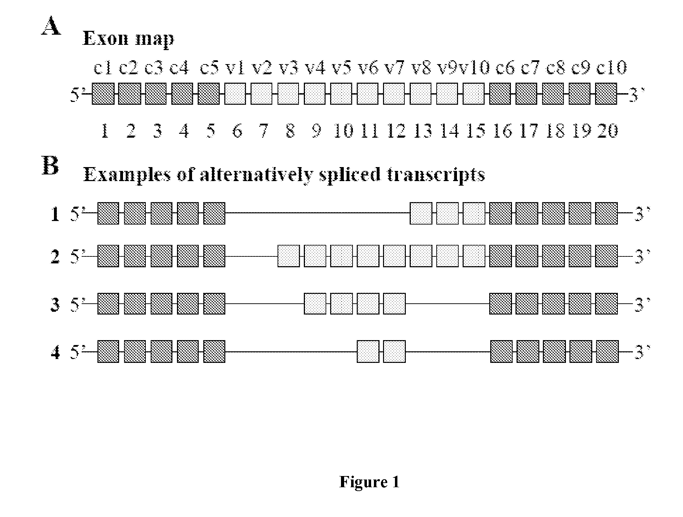

CD44, the main cellular receptor of hyaluronic acid (a glycosaminoglycan that is a major component of the extracellular matrix), is an 80 kD, highly glycosylated transmembrane molecule, with multiple variant isoforms generated by alternative RNA splicing (see Figure 1). It appears to be expressed by most of tumor-initiating cells populations in solid tumors.

However, despite the fact that the population of breast tumor-initiating cells has been immediately characterized as expressing the CD44 molecule, this CD44 molecule has never been considered by this research team or others as a potential target for specifically targeting the breast tumor-initiating cells population, but clearly only as a phenotypic marker.

Indeed, none of the previously cited documents describes or suggests that CD44 might be something more than a phenotypic marker. In contrast, Balic et al (11) indicates that CD44 is an adhesion molecule known to be expressed by most cancer cells and also associated to healthy mammary tissue stem cells.

In addition, the results displayed in the article by Al-Hajj et al (1) originally describing the of CD44⁺CD24^{-/low} breast tumor-initiating cells population clearly show that the mere expression of CD44 is not sufficient to confer a tumor-initiating phenotype, since the CD44+CD24+ breast cancer cells do not display this tumor-initiating phenotype.

Moreover, Sheridan et al (14), who studied the invasive properties of CD44⁺CD24^{-/low} breast tumor-initiating cells, clearly indicate that, despite the clear association between the CD44⁺CD24^{-/low} phenotype and the invasive capacities of breast cancer cells, the invasive capacity do not appear to be linked to the functions of CD24 and CD44 molecules.

The same is true for other solid tumors. Although most identified tumor-initiating cells populations express CD44, this molecule has not been suggested as a potential therapeutic target.

In contrast, Verel et al also showed in human head and neck xenografts that a naked anti-CD44v6 antibody (U36) had no activity *in vivo,* but that the linkage of this antibody to a cytotoxic compound permitted to obtain anti-tumor activity in human head and neck xenografts, suggesting that CD44 might be useful as a phenotypic marker to specifically address a cytotoxic compound to such cells, but that a naked anti-CD44 antibody alone would not result in a therapeutic benefit in the case of solid tumors (15).

The only results that have identified the CD44 molecule as a potential target to fight cancer stem cells have been obtained in acute myeloid leukemia (AML) by Florence Smadja-Joffe's team. The results obtained suggest that CD44 protein plays a role in the proliferation of leukemic cells and that its targeting by a murine anti-human CD44 monoclonal antibody (P245 mAb) inhibits tumor proliferation. Indeed, targeting CD44 inhibits acute myeloid leukemia cells proliferation *in vitro,* affects differentiation and apoptosis (16-17) and reverse blockage of differentiation in AML blasts (8).

More recently, this therapeutic approach has been validated in vivo by Jin et al. who showed that administration of P245 mAb to immune-deficient mice transplanted with human AML markedly reduced leukemic repopulation via interference with stem cell nesting and alteration of AML stem cells fate (18).

In patent applications EP1532984 (19) and WO 2005/049082 (20) filed by this team, the inventors show the possibility to use an anti-CD44 antibody to eradicate specifically pathological stem cells in AML, and extend the strategy to cancer in general. The possibility to use the same strategy to treat breast or colon cancer is also suggested, although there is absolutely no support for this extension except the fact that CD44 is expressed by most cancer cells.

However, leukaemia is clearly a quite different cancer type than solid tumors. In particular, the local microenvironment of cancer cells is completely different in a non solid tumor such as leukaemia, in which cancer cells freely circulate and do not form a sort of solid organ, and in solid tumors, in which the tumor forms a complex and organized entity, resulting in complex interactions between various cancer cells types and reduced accessibility of at least some cancer cells to drugs due to their localization in the tumor solid mass.

In addition, as already explained above, more recent publications such as those by Balic et al (11), Sheridan et al (14), or Verel et al (15) would have dissuaded a skilled person to consider using CD44 as a potential target for inhibiting or eliminating breast tumor-initiating cells, since CD44 is also expressed by healthy mammary tissue stem cells and since CD44⁺CD24⁺ breast cancer cells do not display a tumor-initiating phenotype.

Though, the present inventors have surprisingly found that targeting CD44-positive cells could reduce tumor growth and tumor relapse after breast cancer chemotherapy. They also showed that the anti-CD44 antibody induced strong anti-proliferative (or cytostatic) signals in tumor cells, while other additional mechanisms of action such as ADCC or modification of the interaction between CD44 and hyaluronic acid cannot be excluded. In addition, the results suggest that the effect might be epitope dependant and that anti-CD44 antibodies recognizing an epitope situated in the region of the P245 anti-CD44 antibody epitope should be preferred.

Globally, the results indicate that CD44 molecules might be a key-element in the crosstalk pathway between cancer cells and the environment, necessary for T-IC survival and thus opens new therapeutic opportunities. In particular, an innovative concept of therapy may be implemented, based on the inhibition of T-IC which survive to drug-induced toxicity or radiotherapy using an anti-CD44 antibody, preferably after conventional treatment of the bulk tumor, and possibly in combination with another adjuvant treatment.

Such innovative treatments would permit to efficiently prevent relapse and metastasis of solid tumor using a treatment which would much better tolerated by patients than conventional chemotherapy or radiotherapy, which result in significant and sometimes severe adverse effects.

In a first object, the present invention thus relates to the use of an anti-CD44 antibody or an antigen binding fragment thereof for the manufacture of a drug for treating a solid tumor in a patient, in combination with another anti-cancer treatment.

In a second object, the present invention also relates to the use of an anti-CD44 antibody or an antigen binding fragment thereof for the manufacture of a drug for preventing solid tumor relapse in a patient, in combination with another anti-cancer treatment.

In a third object, the present invention also relates to the use of an anti-CD44 antibody or an antigen binding fragment thereof for the manufacture of a drug for preventing solid tumor metastasis in a patient, in combination with another anti-cancer treatment.

In each case, the anti-CD44 antibody or antigen fragment thereof is for use "in combination with" another anti-cancer treatment, meaning that said anti-CD44 antibody or antigen fragment thereof and said other anti-cancer treatment may be administered simultaneously (together or separately) or successively (in any possible order). In some embodiments, a period of simultaneous administration may be observed and one or both of the treatments may also have a period in which it is administered alone. Thus, the global medicament used for treating a solid tumor, preventing solid tumor relapse, or preventing solid tumor metastasis in a patient in the invention may be considered as a kit of parts comprising both an anti-CD44 antibody (or an antigen binding fragment thereof) and another anti-cancer treatment, which may be administered simultaneously, successively and/or separately. In particular, said other anti-cancer treatment may be administered before, after, and/or simultaneously with the anti-CD44 antibody treatment. Advantageously, said other anti-cancer treatment may be administered before and/or simultaneously with the anti-CD44 antibody treatment.

In a preferred embodiment, said other anti-cancer treatment has been administered before the anti-CD44 antibody treatment, and the patient to which is administered the anti-CD44 antibody treatment has thus been previously treated by another anti-cancer treatment. The other, previously administered, anti-cancer treatment may or not be continued during the administration of the anti-CD44 antibody or antigen binding fragment thereof, although it is preferred that the previous other anti-cancer treatment is stopped.

In another preferred embodiment, the anti-CD44 antibody or antigen binding fragment thereof treatment is administered simultaneously with the other anti-cancer treatment.

By "another anti-cancer treatment" is meant any other suitable treatment approved for cancer treatment. In particular, said other anti-cancer treatment may be selected from the group consisting of chemotherapy, surgery, radiotherapy, hormonotherapy, and/or immunotherapy.

In the case of chemotherapy, at least one chemotherapeutic agent may be used selected from the group consisting of growth inhibitory agents (defined as compounds or compositions which inhibit growth of a cell, either in vitro or in vivo) and cytotoxic agents (defined as compounds or compositions which inhibits or prevents the function of cells and/or causes destruction of cells).

In a particular embodiment of the invention, chemotherapeutic agents are selected from the group consisting of: taxanes, topoisomerase II inhibitors, DNA alkylating agents, anti-metabolite, anti-tubuline, vinca alkaloids, intercalating agents and platinium salts.

In a more particular embodiment of the invention, chemotherapeutic agents are selected from the group consisting of: paclitaxel, docetaxel, doxorubicin, epirubicin, daunorubicin, etoposide, bleomycin, tamoxifen, prednisone, dacarbazine, mechlorethamine, cisplatin, methotrexate, 5-fluorouracil, anthracyclines, adriamicin, vincristine, vinblastine, etoposide, doxorubicin, melphalan, mitomycin C, chlorambucil, daunorubicin, cyclophosphamide and platinium salts.

Of course, any suitable combination of chemotherapeutic drugs may be used, depending on the type of cancer.

As used here, the term "antibody" includes monoclonal antibodies, polyclonal antibodies, bi-specific, multi-specific, antibodies (e.g., bispecific antibodies); human, humanized, chimeric antibodies, single chain antibodies, Fab fragments, F(ab')2 fragments, disulfide-linked Fvs, intrabodies and fragments containing either a VL or VH domain or even a complementary determining region (CDR) that specifically binds to the biologically active CD44 region retaining the P245 antitumoral activity described in the invention. Preferably, the anti-CD44 antibody used in the invention is a monoclonal antibody, or an antigen binding fragment thereof.

The antibody used in the invention may have been modified is several manners. In particular, when the anti-CD44 antibody is of non human origin (such a rabbit, mouse, rat or any other non human species), it is preferable for administration to a human patient that said antibody has been modified to reduce its immunogenicity in humans. Thus, the anti-CD44 antibody may advantageously be a chimeric, human or humanized antibody

Variants of antibodies (including variants of antigen binding site), such as chimeric antibodies, humanized antibodies, veneered antibodies, and engineered antibodies that have the same specificity are also included in the scope of the invention.

Chimeric antibodies are 65-90% human nucleotides sequences and consist of the murine variable regions, which bring about antigen recognition, fused to the constant or effector part of a human antibody. Humanized antibodies are about 95% human, and are made by grafting only the hyper- variable region, or complementarity-determining regions, of the murine antibody - which determines antibody specificity - onto a human antibody backbone.

The developments of genetically engineered transgenic mice and advances in the generation of synthetic human antibody libraries have enabled the production of fully human antibodies on a commercial scale. Chimeric, humanized, and fully human antibodies have less immunogenicity than early antibody constructs and allow repeated antibody administration, an improved capacity to recruit cytotoxic cells and complement, and an increased stability in the circulatory system. These improvements have contributed to the increased therapeutic efficiency of monoclonal antibodies.

The antibody or antigen binding fragment thereof may also have been modified at the level of glycosylation, fucosylation or any other modification of carbohydrate groups corresponding to post-translational modifications, or chemically modified (e.g., by lipophilic anchors).

In contrast, although it is possible that the antibody or antigen binding fragment thereof be further conjugated to another compound, it is preferred that it is not the case.

As used herein, the term "antigen binding fragment" applied to an antibody refers to a portion of an intact antibody which retains the desired biological activity of binding to the antigen recognized by the complete antibody. Examples of antigen binding fragment include, but are not limited to, single chain antibodies, Fab fragments, F(ab')2 fragments, disulfide-linked Fvs, intrabodies and fragments containing either a VL or VH domain or even a complementary determining region (CDR) that specifically binds to the biologically active CD44 region obtained by molecular biology or enzymatic technologies. In some embodiments, said antigen binding fragment is a Fab fragment, a F(ab')2 fragment, a disulfide-linked Fv, or a ScFv fragment.

Small antibody fragments have also been engineered to improve antibody penetration into bulky avascular tumours, especially solid tumours. A single- chain fixed-variable (Fv) region consists of only one heavy-chain variable domain and one light-chain variable domain, which are covalently linked by a short peptide linker.

Antibodies have also been designed that have two different antigen-binding arms and hence have dual-binding specificity ("bispecific antibodies"). An example is when one arm binds to the tumour cell and the other binds to the effector cell. Another example is when the arms bind not only to two different antigens on the tumor cell, but also to the effector cell by their Fc fragment. These constructs increase antibody-mediated tumour-cell killing through the recruitment of host immune-effector cells (T cells, natural-killer cells, and macrophages).

The results obtained by the inventors show that not all anti-CD44 antibodies permit to prevent solid tumor relapse, and suggest that the efficiency of a particular anti-CD44 antibody depends on the epitope recognized by this antibody in the CD44 molecule.

CD44, the main cellular receptor of hyaluronic acid (a glycosaminoglycan that is a major component of the extracellular matrix), is an 80 kD, highly glycosylated transmembrane molecule, with multiple variant isoforms generated by alternative RNA splicing. The CD44 gene is composed of 20 exons. All isoforms comprise a set of exons known as constant exons corresponding to exons n°1-5 and 16-20, and may further comprise one or more exons known as variant exons corresponding to exons n°6-15. Various isoforms comprising variant exons have been described as associated to tumor cells (see Figure 1).

In contrast, the P245 anti-CD44 antibody, which is efficient in the invention for preventing breast tumor relapse after chemotherapy, binds to a region of the CD44 molecule situated in constant exons. More precisely, this epitope is located inside the HA (hyaluronic acid) to CD44 bonding domain, which is situated in a region corresponding to amino acids 12-101 of CD44 antigen isoform 1 precursor (the longest isoform with 742 amino acids) defined by Genbank accession numbers NM_000610.3 (nucleic acid) and NP_000601 (protein). This region corresponds to SEQ ID NO: 1 :

LCLVPLSLAQIDLMITCRFAGVFHVEKNGRYSISRTEAADLCKAFNSTLPTM
AQMEKALSIGFETCRYGFIEGHVVIPRIHPNSICAANN (SEQ ID NO:1)

Thus, in a preferred embodiment, the anti-CD44 antibody or antigen binding fragment thereof used in the invention is specifically directed to an epitope located in SEQ ID NO:1.

However, other antibodies recognize for example a CD44 isoform and bind to regions encoded by non constant regions (21), and other regions of the CD44 molecule may also be suitable as binding regions for an anti-CD44 antibody to obtain the effect of the invention. The scope of the present invention is thus not limited to anti-CD44 antibodies that bind to the CD44 molecule in the same regions than the P245 antibody but rather extends to any anti-CD44 antibody or antigen binding fragment thereof that has the capacity of preventing tumor relapse. The capacity of a given anti-CD44 antibody to prevent tumor relapse may be easily tested by a skilled person using the experiments described in the Examples of the present application.

The nucleic sequences of the P245 anti-CD44 antibody heavy and light chains have been disclosed and are respectively represented by SEQ ID NO:2 and SEQ ID NO:3:

Murine P245 antibody heavy chain
QVQLQQSGDELVRPGSSVKISCKASGYAFSRYWMNWVKQRPGQGLEWIG
QIYPGDGDTNYNGKFKGKATLTADKSSSTAYMQLNSLTSEDSAVYFCARRRWSD
YFGMDYWGQGTSVTVSSAKTTPPSVYPLAPGSAAQTNSMVTLGCLVKGYFPEPVT
VTWNSGSLSSGVHTFPAVLESDLYTLSSSVTVPSSPRPSETVTCNVAHPASSTKVDK
KIVPRDCGCKPCICTVPEVSSVFIFPPKPKDVLTITLTPKVTCVVVDISKDDPEVQFS
WFVDDVEVHTAQTQPREEQFNSTFRSVSELPIMHQDWLNGKEFKCRVNSAAFPAPI
EKTISKTKGRPKAPQVYTIPPPKEQMAKDKVSLTCMITDFFPEDITVEWQWNGQPA
ENYKNTQPIMNTNGSYFVYSKLNVQKSNWEAGNTFTCSVLHEGLHNHHTEKSLSH
SPGK (SEQ ID NO:2)

Murine P245 antibody light chain
DVVMTQTPLTLSVTIGQPASISCKSSQSLLHSNGKTYLNWLLQRPGQSPKLLI
YLVSKLESGVPDRFSGSGSGTEFTLKISRVEAEDSGVYYCLQATHFPLTFGAGTKLE

LKRADAAPTVSIFPPSSEQLTSGGASVVCFLNNFYPKDINVKWKIDGSERQNGVLN
SWTDQDSKDSTYSMSSTLTLTKDEYERHNSYTCEATHKTSTSPIVKSFNRNEC
(SEQ ID NO:3)

Although other parts of an antibody variable domain may be in contact with the antigen, it is well known that the 3 loops of both the heavy and light chains corresponding to complementary determining regions (CDR) 1, 2 and 3, and especially the CDR3 region, are the main regions involved in antigen recognition.

Since the P245 anti-CD44 antibody has been found efficient in vivo by the inventors, it is advantageous that the anti-CD44 antibody used in the invention displays the same CDR1, CDR2, and CDR3 regions of the heavy and light chain as the P245 antibody. Thus, in a preferred embodiment, the anti-CD44 antibody or antigen binding fragment thereof used in the invention has a heavy chain comprising CDR1, CDR2, and CDR3 regions represented by GYAFSRYW (SEQ ID NO:4), IYPGDGDT (SEQ ID NO:5), and ARRRWSDYFGMDY (SEQ ID NO:6) respectively, and a light chain comprising CDR1, CDR2, and CDR3 regions represented by QSLLHSNGKTY (SEQ ID NO:7), LVS, and LQATHFPLT (SEQ ID NO:8) respectively.

In addition, two IgG1 or IgG4 isoforms of a chimerized P245 antibody with the murine variable domain of the P245 antibody and human IgG1 or IgG4 constant domains have been described (20). In a preferred embodiment, the anti-CD44 antibody used in the invention is selected from the group consisting of the P245 murine antibody which has a heavy chain represented by SEQ ID NO:2 and a light chain represented by SEQ ID NO:3; an IgG1 chimeric P245 antibody which has a heavy chain represented by SEQ ID NO:9 and a light chain represented by SEQ ID NO:10; or an IgG4 chimeric P245 antibody which has a heavy chain represented by SEQ ID NO:11 and a light chain represented by SEQ ID NO:10:

Chimeric P245 antibody heavy IgG1 chain
QVQLQQSGDELVRPGSSVKISCKASGYAFSRYWMNWVKQRPGQGLEWIG
QIYPGDGDTNYNGKFKGKATLTADKSSSTAYMQLNSLTSEDSAVYFCARRRWSD

YFGMDYWGQGTSVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEAL
HNHYTQKSLSLSPGK (SEQ ID NO:9)

Chimeric P245 antibody light kappa chain
DVVMTQTPLTLSVTIGQPASISCKSSQSLLHSNGKTYLNWLLQRPGQSPKLLI
YLVSKLESGVPDRFSGSGSGTEFTLKISRVEAEDSGVYYCLQATHFPLTFGAGTKLE
LKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
(SEQ ID NO:10)

Chimeric P245 antibody heavy IgG4 chain
QVQLQQSGDELVRPGSSVKISCKASGYAFSRYWMNWVKQRPGQGLEWIG
QIYPGDGDTNYNGKFKGKATLTADKSSSTAYMQLNSLTSEDSAVYFCARRRWSD
YFGMDYWGQGTSVTVSSASTKGPSVFPLAPCSRSTSESTAALGCLVKDYFPEPVTV
SWNSGALTSGVHTFPAVLQSSGLYSLSSVVTVPSSSLGTKTYTCNVDHKPSNTKVD
KRVESKYGPPCPSCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKVSN
KGLPSSIEKTISKAKGQPREPQVYTLPPSQEEMTKNQVSLTCLVKGFYPSDIAVEWE
SNGQPENNYKTTPPVLDSDGSFFLYSRLTVDKSRWQEGNVFSCSVMHEALHNHYT
QKSLSLSLGK (SEQ ID NO:11).

The inventors have demonstrated the efficiency of the method for breast cancer. However, since solid tumors share a number of features in term of tumor organization and constraints, and since it appears that tumor-initiating cells of other solid tumors than breast cancer also express CD44, the present invention may be used for solid tumors in general. However, the invention is particularly useful in sequential combination of standard therapy followed by anti-CD44 antibody (or antigen binding fragment thereof) treatment, in any clinical situation in which a strong response is expected from standard therapy, and in which, nevertheless, relapses are frequent. Non limitative examples of such solid tumors for which the invention may be used include breast cancer, prostate cancer, colon cancer, head and neck cancer, ovary cancer, melanoma, small cell lung cancer, testis cancer, kidney cancer, pancreatic cancer and osteosarcoma.

In the invention, the anti-CD44 antibody may be administered at a dose of less than 150 mg/kg/j. The anti-CD44 antibody may also be administered at doses of at least 20 mg/m2, with escalated doses in steps of 20 mg/m2.

The results obtained by the inventors with the P245 antibody show a cytostatic effect and not a complete elimination of the tumor-initiating cells. Thus, it is preferred that the anti-CD44 antibody is continuously administered to the patient treated, to prevent that relapse occurs after the end of anti-CD44 antibody administration.

In addition to the combination with another anti-cancer treatment, the anti-CD44 antibody treatment may be further administered in combination with a third compound which is an anti-angiogenic agent. This may be especially useful when the invention is used to prevent solid tumor relapse since angiogenesis is necessary to fully restore a solid tumor. In particular, when the anti-CD44 antibody treatment is administered after another conventional anti-cancer treatment such as for instance surgery, chemotherapy or radiotherapy, the anti-CD44 antibody treatment may be further administered in combination with an anti-angiogenic agent. Suitable anti-angiogenic agents include an anti-VEGF antibody, an anti-VEGF receptor antibody, thalidomide, and SU 11657 (Sutent).

The invention also concerns a method for treating a solid tumor in a patient in need thereof, comprising administering to said patient a combination of an effective amount of an anti-CD44 antibody and of another anti-cancer treatment.

The invention also relates to a method for preventing solid tumor relapse in a patient, comprising administering to said patient an effective amount of an anti-CD44 antibody, wherein said patient is simultaneously treated or has been previously treated by another anti-cancer treatment.

The invention also relates to a method for preventing solid tumor metastasis in a patient, comprising administering to said patient an effective amount of an anti-CD44 antibody, wherein said patient is simultaneously treated or has been previously treated by another anti-cancer treatment.

The invention also relates to a method for inhibiting solid tumor initiating cells growth and/or invasiveness in a patient, comprising administering to said patient an effective amount of an anti-CD44 antibody, wherein said patient is simultaneously treated or has been previously treated by another anti-cancer treatment.

In any method of treatment according to the invention, all particular embodiments described previously may be used.

### DESCRIPTION OF THE DRAWINGS

**Figure 1****. Exon organization of CD44.** (A) Map of CD44 exons. The dark grey squares represent the constant-region exons, while the light grey represent the variant exons. (**B**) Examples of alternatively spliced transcripts: 1) epithelial CD44 (CD44v8-v10); 2) keratinocyte CD44 (CD44v3-v10); 3) pMeta-1 (CD44v4-v7); 4) pMeta-2 (CD44v6, v7).

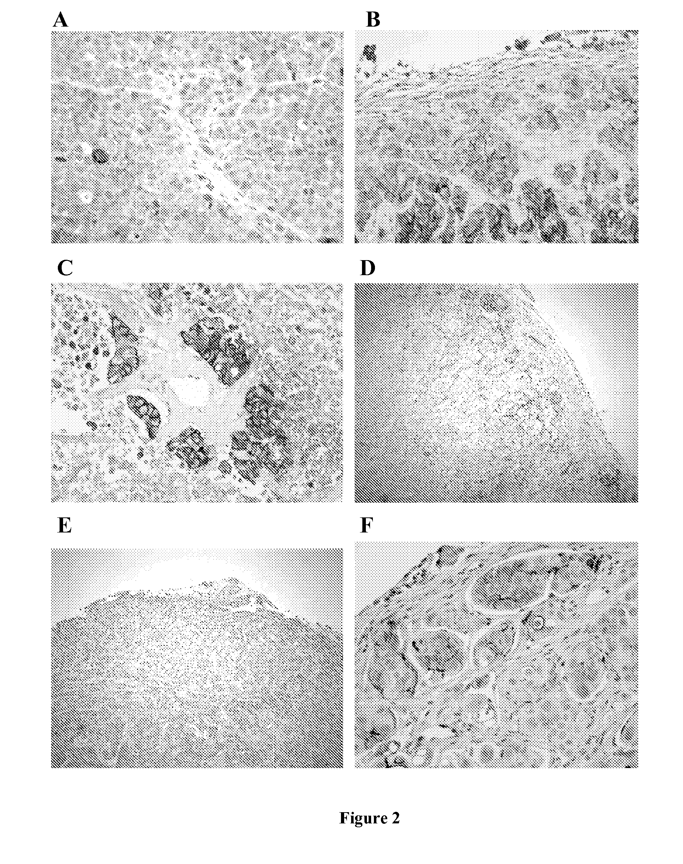

**Figure 2****.** Immunohistochemical staining of three HBCx showed a heterogeneous staining of CD44. **A:** HBCx-8 CD44 positive cancer cell isolated in the tissue (not treated). The staining is membranar and irregular. **B:** A gradient of staining is observed from the periphery to the center areas (untreated HBCx-8 tumor). **C:** HBCx-3 tumor (untreated) Strong staining if islets of survival cells in the necrotic areas, at the vicinity of blood vessels **D:** HBCx-3 (untreated) Low staining of CD44 positive cells in the periphery of the tumor. Note the lack of necrosis and of CD44 positive cells in the central area, usually present in not treated tumors. **E:** large tumor section weakly or not stained and small cellular islets with stained bounds **F:**or HBCx-3 treated with p245 anti-CD44 antibody.

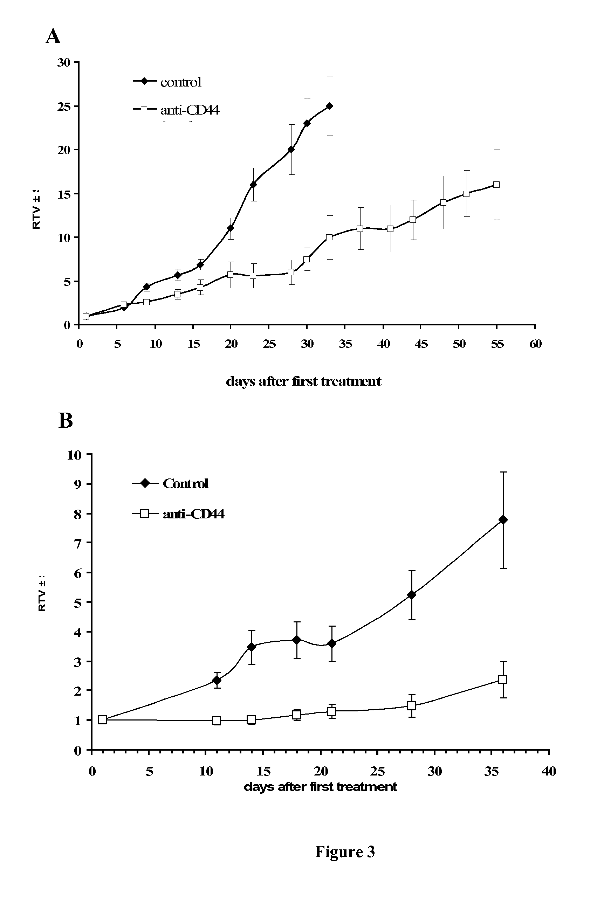

**Figure 3****.** Antitumoral effects of P245 mAb on the growth of HBC xenografts **A.** Growth curves of HBCx-8 as a function of time, untreated (full plots) or treated (empty plots) with P245 mAb anti-CD44 antibody. **B.** Growth curves of HBCx-3 (ER+) as a function of time, untreated (full plots) or treated (empty plots) with P245 anti-CD44 antibody.

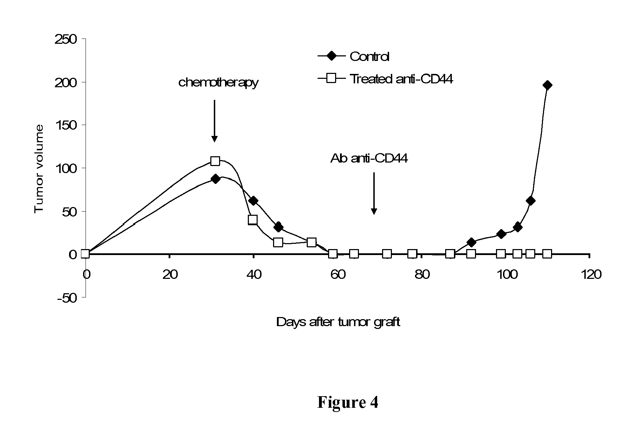

**Figure 4**. Effect of the anti-CD44 P245 mAb treatment on the relapse of HBCx-10 after a pre-treatment with adriamycin/cyclophosfamide which led to complete tumor regression. Tumor bearing mice were all treated with the adriamycin/cyclophosfamide combination (AC) treatments (adriamycin 2 mg/kg, cyclophosphamide 100 mg/kg) and then separated in two groups when tumors remission was complete, with tumors not palpable anymore. One group was treated three times per week with P245 mAb 3mg/kg for 5 weeks. At this time the control group showed 16 of 21 tumors (76 %) relapsed, whereas only 4/13 tumors (30%) of the treated group had relapsed. Thus, P245 mAb treatment prevent tumor relapse after chemotherapy remission.

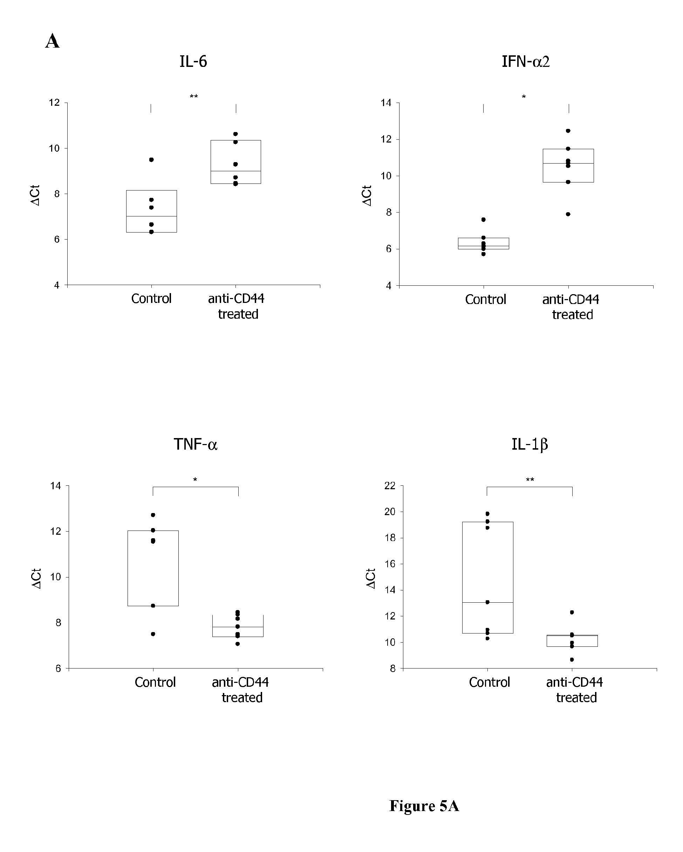

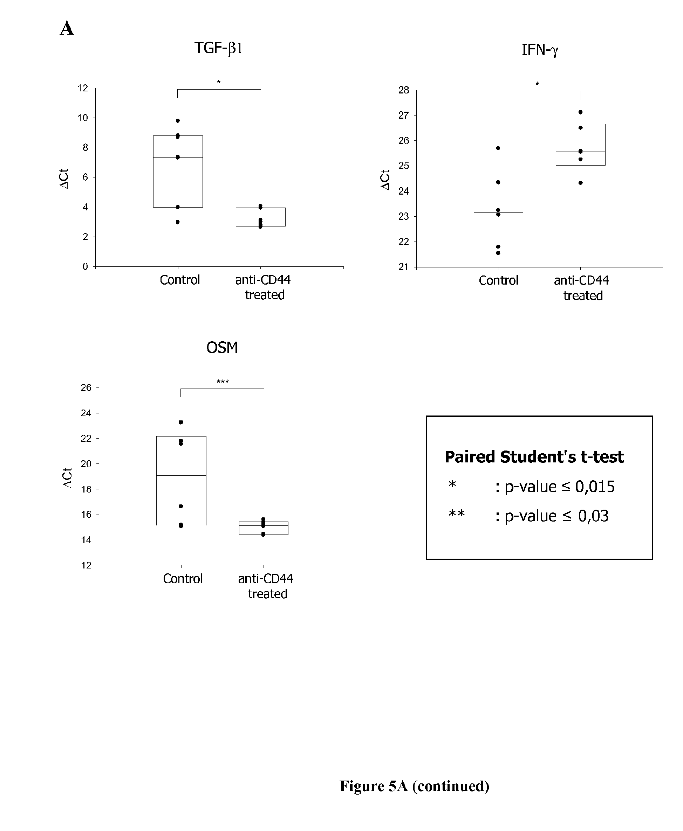

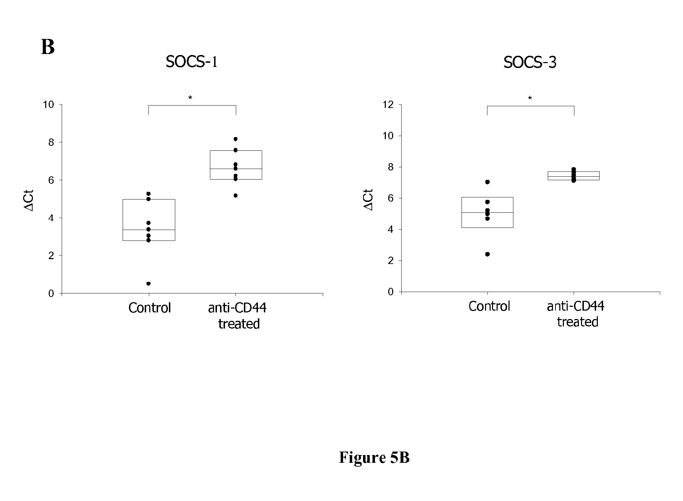

**Figure 5****.** mRNA expression of selected cytokines (**A**) and SOCS (**B**) by Taqman gene expression assay. Taqman results are shown as DCt values between each test gene and PBGD housekeeping gene. Each tumor sample is represented by a black point (•). Dispersion is shown by box plot graph. The boundary of the box closest to zero indicates the 25^{th} percentile, a line within the box marks the median value, and the boundary of the box farthest from zero indicates the 75^{th} percentile. Fold increase values are calculated. The paired Student's t-test was used to test the mean difference between control and treated groups.

### EXAMPLES

### EXAMPLE 1. Inhibition of tumor growth and prevention of post-chemotherapy relapse in human breast cancer xenografts by administration of the anti-CD44 P245 monoclonal antibody

### 1.1. Patients

HBCx (human breast cancer xenografts) originated from HBC (human breast cancer) of 3 different patients, with consent. All had benefit of tumorectomy (or mammectomy), all were of grade III, were large tumors (T3), none had metastases. The examination of lymph node detected tumor invasion in the patient HBC-3, and none in the two other patients. None overexpressed HER2 or progesterone receptors, only HBCx-3 had positive estrogen receptors. The patients HBC-3 and HBC-10 received an adjuvant chemotherapy, based on anthracyclin and radiotherapy. The follow-up of patients HBC-3 and HBC-8 showed a disease-free interval of 27 and 22 months, respectively, interrupted by local recurrence of the tumor. The patient HBC-10 is disease-free since 32 months (Table 1).

**Table 1: Clinical information related to the original patient tumors and growth characteristics of the corresponding xenografts.**

| Tumor Code HBCx | Histology Grade Stage | Markers | Treatment | Time to progression (DFS in months) | Xenograft Latency° (days) | Xenograft Doubling time* (days) | P53 status^{$} | % CD44 positive cells°* |
|---|---|---|---|---|---|---|---|---|
| HBCx-3 | Ductal Grade III T3N+M0 | ER+, PR-HER2- | Surgery FEC Rx | 27 months local recurrence | 21 | 17 | Mutated | 30 |
| HBCx-8 | Ductal Grade III T3N0M0 | ER-, PR-, HER2- | Surgery | 22 months local recurrence | 21 | 10 | Wild type | 11 |
| HBCx-10 | Ductal Grade III T3N0M0 | ER-, PR-, HER2- | Surgery AC Rx | > 32 months | 26 | 17 | Mutated | 30 |

DFS: disease free survival. ER, estrogen receptor, PR, progesterone receptor, HER2 also designated as ErbB2 oncogen, FEC is a combination of 5-fluorouracile, epirubicin and cyclophosfamide, AC is a combination of adriamycin and cyclophosfamide ° Mean time in days between the graft and palpable tumor formation. * Mean time in days between 2-fold measures in exponential growth phase. ^{$}As reported in Marangoni et al, with permission. °*, as measured by FACS analysis.

### 1.2. Material and Methods

### 1.2.1. Animals and establishment of tumor xenografts

HBC xenografts were established. Briefly, breast cancer fragments were obtained from patients at the time of surgery, with informed written patient consent for research use. Fragments of 30-60 mm3 were grafted subcutaneously into the interscapular fat pad of 8-12 weeks old female Swiss nude mice, under avertin anesthesia. Mice were maintained in SPF animal housing (at the Curie Institute) and received estrogen (17 mg/ml) diluted in drinking water. Tumors appeared at the graft site 2 to 8 months after grafting. They were subsequently transplanted from mouse to mouse, and stocked frozen in DMSO- fetal calf serum solution, or frozen dried in nitrogen for further studies, and a fragment fixed in PBS-formol for histological studies. The experimental protocol was approved by the Ile de France-I ethical committee, according to French regulations.

### 1.2.2. Compounds and therapeutic assays

Adriamycin, 2 mg/kg (Doxorubicin®, Teva Pharmaceuticals, France) and cyclophosfamide, 100 mg/kg (Endoxan®, Baxter, France) diluted in 0.9% NaCl were given by the intraperitoneal route (i.p.) at 3 week intervals, for two cycles of treatment.

HBCx were transplanted into female 8-week old Swiss nude mice, as described above, one tumor being transplanted into 8 recipients. When tumors reached a volume of 60-200 mm3, mice were individually identified and randomly assigned to the control or treated groups (eight to 12 mice per group), and the treatments were started.

### 1.2.3. Evaluation of antitumoral efficacy of compounds

Tumor growth was evaluated by measurement of two perpendicular diameters of tumors with a caliper, 2 to 3 times per week. Individual tumor volumes were calculated as V = a x b2/2, a being the largest diameter, b the smallest. For each tumor, Vs were reported to the initial volume, as relative tumor volume (RTV). Means (and standard errors) of RTV in the same treatment group were calculated, and growth curves were established as a function of time. Optimal tumor growth inhibition (TGI) of treated tumors versus controls was calculated as the ratio of the mean RTV in treated group (T) to the mean RTV in the control group (C) at the same time. Statistical significance of TGI was calculated by the paired Student's *t* test, by comparing the individual RTVs in the T and C groups. T/C tumor growth delays (GD) were calculated as the time required to reach the same RTV in the treated group T and the control group C (usually at a RTV of 4). Mice were ethically sacrificed when the tumor volume reached 2500 mm3.

### 1.2.4. Antibodies preparation for treatment.

### P245 and Hermès-3 hybridomas were supplied by Laurence Boumsell (MAT -

Evry) and David Naor (Lautenberg Center for General and Tumor Immunology, Jerusalem) respectively. In summary, the hybridomas were adapted in serum-free culture and P245 and Hermès-3 mAbs were produced in vitro using CELLine CL1000 bioreactors (VALDEA Biosciences). Highly concentrated titers of antibodies were collected twice a week. Purification of hybridomas supernatant was typically performed on an AKTA purifier system (GE Healthcare) by affinity chromatography using a HiTrap protein G Sepharose column. Fractions containing antibody were pooled and subjected to buffer exchange against phosphate-buffered saline solution (PBS) with a HiPrep 26/10 desalting column. Sterile-filtered antibody batches were quantified by Bio-Rad DC Protein Assay Kit and purification process was validated by SDS-PAGE stained with silver nitrate. Finally P245 and Hermès-3 mAbs functionality was assessed by binding tests developed by Lecomte and colleagues: in vitro binding on Fc-CD44 fusion protein and binding to stable Jurkat T cell CD44 transfectants by flow cytometry (FACS Calibur).

IgG isotype control from murine myeloma were purchased as clarified ascites fluid from Sigma-Aldrich, purified and assessed as described above.

Murine P245 and Hermes-3 mAbs or murine IgG control were intraperitoneally injected at a dosage of 3 mg/kg per injection three times a week for 4 to 8 weeks, as indicated.

### 1.2.5. Cell staining, sorting and flow cytometry.

Tumors were removed from mice and tumor cells were extracted by gentle mechanical crushing and filtration through two layers of sterile cotton gauze and enzymatic (0.05%trypsin/0.025% EDTA) digestion according to Clarke procedure (1). Single cells were washed once with 1% calf fetal serum/phosphate-buffered saline (PBS) and suspended in PBS, 10⁶cells/ml. FITC fluorochrome-conjugated P245 mAb and IgG1 control were prepared and added to the cell suspension at the concentration of 1µg / 1.10⁵ cells / 200µL and incubated at 4°C for 40 min in the dark. The labeled cells were washed in PBS then analyzed on a FACS-vantage (BD Biosciences).Death cells were eliminated using DAPI exclusion.

### 1.2.6. Histology of xenografts

Tumors were removed from mice and immediately fixed in a 10% formol solution for immunohistological analysis. Biotin-conjugated P245 and Hermès-3 antibodies were prepared according to manufacturer's instructions (Pierce). Labeled antibodies functionality was assessed by binding to stable Jurkat T cell CD44 transfectants revealed with APC-streptavidin by flow cytometry (FACS Calibur).

### 1.2.7. Gene expression and signaling pathways exploration

Specific gene expression and signaling pathways induced by CD44 targeting were investigated on treated and control tumours frozen in liquid nitrogen and stored at -80°C until extraction. Frozen tumour pieces were ground into powder using a mortar and pestle in the presence of liquid nitrogen. For protein extraction, a total of 100 to 200mg tumor powder were dissolved in TCA 10% and precipitated at 4°C overnight and spun down twice by centrifugation at 15000 x g. Pellets were finally resuspended at a concentration of about 20mg/mL in Lithium dodecyl sulfate (LDS) reduced buffer containing proteases inhibitors. Western blotting were performed with 18-wells precast 7,5 or 12% Tris-HCl Criterion electrophoresis gels using the Biorad Criterion system, and quantified by densitomeric analysis with ImageJ software. Apoptosis markers were tested woth specific antibodies to pro and cleaved caspase -3, -8, -9 and PARP. Several signaling pathways were explored with antibodies to phospho and total IGF1R, ERK1-2, P38 MAPK, Akt, STAT3, STAT5, ERM (Ezrin -Radixin - Moesin) Merlin. All antibodies were purchased to Cell Signaling Technology.

For mRNA extraction, 50mg tumour powder were suspended in 1 ml Trizol reagent (Invitrogen) and treated according to the manufacturer's instructions. cDNA synthesis was performed with MluV reverse transcripase (Roche). Quantitative real-time PCR was performed by TaqMan® Gene Expression Assays either with Assays-on-Demand (Applied Biosystems) or with sequence specific TaqMan primers and probes designed by using the PrimerExpress Software. Assays were realized with TaqMan universal PCR master mix following the manufacturer's instructions (Applied Biosystems) on an ABI PRISM 7700 Sequence Detection System (Applied Biosystems). The relative RNA expression levels were calculated via comparative Ct method, using the porphobilinogene deaminase gene (PBGD) as reference gene. We chose to investigate gene expression of TGF-β1, Oncostatin M, TNF-α, IL-1β, γ- and α2 interferons, Interleukin-6, -8, GMCSF proinflammatory cytokines as well as SOCS family members (SOCS-1, -2, -3, CISH) and p21, p27, cyclin D 1 cell cycle regulatory proteins.

### 1.3. Results

### 1.3.1. HBCx xenograft models, frequency and tissular distribution of CD44 positive cells HBC xenografts.

Three different HBCx were used (see paragraph 1.2.1), they were obtained at tumorectomy or mammectomy (Table 1). The mean latency of tumor appearance after implantation is of 3-4 weeks and their doubling time of 10-17 days. P53 was not mutated in HBCx-8, and mutated in the two others.

FACS analysis showed that the fraction of CD44 positive cells was of 11, 30 and 50% in HBCx-8, HBCx-10 and HBCx-3, respectively. Immunohistochemical staining of the three HBCx showed a heterogeneous staining of CD44.

In HBCx-8 few isolated strongly stained cells were observed (Figure 2A) as well as an increased gradient of positivity toward the central tumor area (Figure 2B).

In HBCx-3, that presents necrotic areas as tumors grow up to 500mm3, a very strong CD44 staining was found in cancer cells islets cells surviving in necrosis, at the vicinity of blood vessels (Figure 2C). Immunohistochemistry also showed a peripherical CD44 staining oftumoral acini.

### 1.3.2. Antitumoral effects of P245 mAb on the growth of HBC xenografts

To evaluate the antitumor activity of The P245 mAb, tumor growth was evaluated in two HBCx models. The P245 mAb treatment was started 4 weeks after tumor graft, when the tumor sizes were approximately of 5 x 5 mm. (63 mm3). With the HBCx-8 model, (Figure 3A), 4 weeks after start of treatment tumor growth inhibition was of 70% in the P245 mAb-treated group (p = 0.01) and the tumor growth delay at the end of treatment (60 days) was of 30 days, 1.5 fold as compared to control. After arrest of treatment, at day 55, an acceleration of tumor growth of the treated group was noticed, suggesting that P245 mAb treatment should be administrated continually to inhibit tumor proliferation. With the HBCx-3 model (ER positive), the tumor growth was completely stabilized by P245 mAb treatment (Figure 3B).

At the end of treatment, five mice bearing the HBCx-3 and HBCx-8 were sacrificed in each group. Residual tumors were fixed for histological analysis or frozen for protein extraction and western blotting. No apparent necrosis neither apoptotic cells were found in P245 mAb treated tumors. The percent of cells with Ki67 antibody staining (proliferative cells) was not found to be different in control and in treated group, despite a large difference in the tumor size. After P245 mAb treatment, the residual tumors HBCx-3 showed a low staining in the peripheral layers, not present in the control group (Figure 2D).

### 1.3.3. Proinflammatory cytokines expression induction by P245 mAb-treatment in HBC xenografts

Except its role in adhesion and migration process, CD44 is known to induce synthesis of many cytokines and chemokines in macrophages. In Acute Myeloid Leukemia it has been shown that, in leukemic blasts, CD44 ligation with specific anti-CD44 mAbs induces a burst of differentiating cytokines, as IL-6 and GM-CSF, locally-acting through an autocrine / paracrine pathway. Here the inventors focused our gene expression assays on proinflammatory cytokines signaling in 14 P245 mAb treated versus untreated HBCx-8 tumour samples (40 days treatment). Significantly they showed a marked increase in mRNA levels of TGF-β1, oncostatin M, TNF-α, and IL-1β (fold-changes: 14 - 8 - 8 and 20 respectively, Figure 5). Interestingly significant decrease of SOCS-1 and SOCS-3 were observed in treated tumours (fold-changes: 9 and 3 respectively, see Table 2). No statistically significant changes in mRNA levels were shown for p27, p21 and cylin D1 cell cycle regulators proteins or other tested cytokines. The results show for the first time that a specific anti-CD44 mAb can induce proinflammatory cytokines in breast cancer CD44-positive tumor cells.

**Table 2. Fold increase or decrease in treated samples compared to control samples, calculated as: FI = 2-DDCt (User Bulletin #2, ABI Prism 7700 Sequence Detection System (Applied Biosystems)).**

| Gene | Fold increase (P245m-treated / Control) |
|---|---|
| TGF-β1 | 14 |
| TNF-α | 8 |
| IL-1β | 20 |
| Oncostatin M | 8.3 |
| IFN-γ | -3 |
| SOCS-1 | -9 |
| SOCS-3 | -3 |
| IL-6 | -2.6 |
| IFN-α2 | -16 |

### 1.3.4. Signalling pathways exploration by western blotting

In recent works it has been showed that CD44 targeting by specific mAbs could induce apoptosis. In 14 tumor protein extracts from P245 mAb treated versus untreated HBCx-8-xenografted mice no cleaved apoptosis markers were detected after 40 days treatment as confirmed by immunohistochemical staining. Among all screened signalling pathways, decrease in p38 phosphorylation was shown in treated tumors (data not shown). High heterogeneity within ERM phosphorylation status was found without statistical correlation in P245 mAb treatment response.

### 1.3.5. Inhibition of tumor relapse of HBCx-10 after chemotherapy

The cancer stem cell hypothesis raises the existence of a drug-resistant small subpopulation that should be responsible for relapse after a chemotherapy-induced remission. The HBCx-10 xenograft is exquisitely sensitive to adriamycin/cyclophosfamide (AC) treatment but shows tumor relapse after apparent complete remission. As the tumors reached a mean volume of 100 mm3 (range of 63-250 mm3), all mice received an AC injection which led to complete regression of all tumors. The group was shared in two, one being without treatment, one receiving P245 mAb injections. Tumor regrew in 76% of control mice (16/21) after several weeks. Only 4/13 regrew in the P245 mAb-treated group. As for the tumor growth assays, when the P245 treatment is stopped, tumor relapsed, suggesting that continually treatment is required to prevent tumor recurrence (Figure 4).

The inhibition of tumor relapse is CD44-related as administration of IgG1 isotype administration does not changed the relapse frequency in the same conditions. Moreover, treatment of mice with Hermes-3 (an anti-CD44 antibody targeting a different epitope) does not reduce relapse frequency.

### 1.4. Discussion

Several studies indicate that solid tumors contain a very small subpopulation of cancer cells with stem-cell properties. Such cancer cells, identified and purified by their CD44 positive profile, had the property to reconstitute a new tumor when re-injected into immunodeficient mice. In breast cancers, it is noticeable that only a small subpopulation of CD44+ cells (CD44+CD24-) had this stem cell property whereas the CD44-CD24- cells or the CD44+CD24+ cells had not (1). Such CD44+ tumor-initiating cells (T-IC) were also found in pancreas, brain, head and neck and colon cancers (1-5, 10).

The resistance of T-IC to stress is illustrated by their resistance to radiation as published by Phillips et al (22). Their multidrug resistance phenotype is suspected and T-IC could be at the origin of relapse after transiently efficient therapies. The role of T-IC in the formation of metastases is suggested by several studies (11, 14, 23-24). T-IC have been isolated from breast cancers and their genomic profile has been studied by microarray (25). A genomic signature has been obtained.

Although T-IC have been shown in several solid tumors as expressing the CD44 molecule, which may be used as a phenotypic marker to identify and purify these cells, the CD44 molecule has only be considered in these publications as a useful phenotypic marker, and not as a potential target molecule for inhibiting or eliminating the T-IC population.

In contrast, several publications concerning breast cancer indicate that CD44 should only be considered as a phenotypic marker. Balic et al (11) indicates that CD44 is an adhesion molecule known to be expressed by most cancer cells and also associated to healthy mammary tissue stem cells.

In addition, the results displayed in the article by Al-Hajj et al (1), who originally described the of CD44⁺CD24^{-/low} breast tumor-initiating cells population, clearly show that the mere expression of CD44 is not sufficient to confer a tumor-initiating phenotype, since the CD44+CD24+ breast cancer cells do not display this tumor-initiating phenotype.

Moreover, Sheridan et al (14), who studied the invasive properties of CD44⁺CD24^{-/low} breast tumor-initiating cells, clearly indicate that, despite the clear association between the CD44⁺CD24^{-/low} phenotype and the invasive capacities of breast cancer cells, the invasive capacity do not appear to be linked to the functions of CD24 and CD44 molecules.

In human head and neck xenografts, Verel et al also showed that a naked anti-CD44v6 antibody (U36) had no activity *in vivo,* but that the linkage of this antibody to a cytotoxic compound permitted to obtain anti-tumor activity in human head and neck xenografts, suggesting that CD44 might be useful as a phenotypic marker to specifically address a cytotoxic compound to such cells, but that a naked anti-CD44 antibody alone would not result in a therapeutic benefit in the case of solid tumors (15).

The only results suggesting that CD44 might be a potential therapeutic target in cancer have been obtained in acute myeloid leukaemia (AML) (8, 16-20).

However, leukaemia is clearly a quite different cancer type than solid tumors. In particular, the local microenvironment of cancer cells is completely different in a non solid tumor such as leukaemia, in which cancer cells freely circulate and do not form a sort of solid organ, and in solid tumors, in which the tumor forms a complex and organized entity, resulting in complex interactions between various cancer cells types and reduced accessibility of at least some cancer cells to drugs due to their localization in the tumor solid mass.

It would thus not be reasonable to extrapolate results obtained in leukaemia to solid tumors based only on the fact that T-IC of solid tumors express CD44.

In addition, as described above, other publications concerning more particularly solid tumors would rather have dissuaded a skilled person to test the ability of anti-CD44 antibodies in the treatment of solid tumors (1, 11, 14, 15).

However, in the present application, the inventors have found that human breast cancers xenografts (HBCx), established into nude mice by direct graft of tumor samples from patients expressed the CD44 epitope, as shown by immunohistochemistry. Interestingly, CD44+ cells are either isolated, very rare in numerous CD44 negative cells. This is in agreement with the observation of other authors. The study also revealed that strongly positive CD44 tumor cells are present as small islets in the necrotic areas of tumors at the contact of capillary blood vessels. Although their function of T-IC is not demonstrated, these CD44+ cells could be associated to survival in stress conditions, and to a contact with blood vessels.

Treatment by intraperitoneal injections of P245 mAb anti-CD44 slowed down the growth of the two tumors tested, HBCx-3 and HBCx-8, as long as the antibody was administered. At the arrest of treatment, the tumors started to grow. The growth was delayed although mitoses were still detectable, and that there was no indication of apoptosis. At histology, the CD44 staining was weak and limited to few groups of cells in the periphery of tumors.

A significant decrease in phosphorylation of the p38 mitogen-activated protein kinase (MAPK) was found in the treated HBCx-8 tumors as compared with control.

Decrease of activation of this p38-MAPKinase negative appears as a consequence of the CD44 blockage by the P245 mAb treatment. P245 mAb treatment increased (8-fold) the mRNA expression of Transforming Growth Factor-beta-1 (TGF-β1), Oncostatin M, Tumor Necrosis Factor-alpha (TNF-α) and Interleukin-1-β (IL-1β, all these cytokines are known as suppressors of tumor growth. In the same way, increase of SOCS-1&3, major suppressors of Cytokine Signalling (SOCS) could re-inforce growth control. Altogether, these results suggest that P245 mAb treatment provokes strong anti-proliferative signals in HBCx tumor cells.

Using *Hermes-3,* another anti-CD44 antibody with different kinetics properties that recognises an epitope of CD44 different of this recognised by the P245 mAb, no regrowth inhibition of HBCx-10 tumors was observed (data not shown). This data is important because it could indicate that the efficacy of the P245 mAb is epitope-dependent. The P245 mAb has been extensively described (see notably ref 20). CD44 is a glycosylated protein which exposed different epitopes (26). Moreover, CD44 is submitted to alternative splicing, generating numerous variants, which all possess exons known as "constant exons" and may or not additionally possess one or more "variant exon(s)". Among these variants, the CD44v6 has been described as frequently produced in tumors. As already mentioned, it has been observed in human head and neck xenografts that a naked anti-CD44v6 antibody (U36) has no activity *in vivo,* but that the linkage of this antibody to a cytotoxic compound showed antitumor activity (15).

It is thus possible that targeting of the CD44 molecule may be efficient for the treatment of solid tumors only if specifically directed to the region in which is situated the epitope recognized by the P245 antibody. This region has been localized in a region corresponding to amino acids 12-101 of CD44 antigen isoform 1 precursor (the longest isoform with 742 amino acids) defined by Genbank accession numbers NM_000610.3 (nucleic acid) and NP_000601 (protein). This region corresponds to SEQ ID NO:1.

Relapse of tumors after therapy is frequent in numerous tumors. In localized breast cancers, neoadjuvant or adjuvant treatment, combined with surgery and radiotherapy cure patients in a large number of cases. Relapses, such as local recurrences or metastases survene in less than 10% with very variable delay. The risk of relapse is associated with the tumor size, the mitotic index, the detection of invaded lymph node, the lack of expression of hormone receptor. In case of metastatic breast cancers, hormonotherapy and chemotherapy delay the outcome of the disease, but without curing the patients.

In this study, tumor relapse was reproduced using the HBCx-10 model. This tumor is a high responder to an adriamycin/cyclophosfamide combination, a standard chemotherapy used in breast cancer treatment. Results show that adriamycin/cyclophosfamide treatment induces the regression of all implanted tumors, but does not cure the mice. In almost all mice, the tumors regrew at the implantation site after a period of apparent remission.

Treatment with the P245 mAb, given to mice while the tumors were no more palpable, reduced the percentage of relapses to 50%. However, when the treatment is stopped, relapse does occur, suggesting that target cells are not eradicated but rather arrested in a reversible way. Such a mechanism of action, called cytostatic, is observed for other antibodies used in cancer therapy (cetuximab, herceptin, ...).

The experiment disclosed in the present application thus revealed the existence of T-IC in the HBCx-10 xenograft, and that T-IC are adriamycin/cyclophosfamide-resistant. They are presumably CD44 positive, because an anti-CD44 antibody reduced their growth whereas the IgG1 isotype control did not.

In addition, the obtained data show that CD44-therapy could increase the benefit of standard chemotherapies in breast cancers, and suggest that it might also have a favourable effect in other solid cancers in which CD44 T-IC have been detected, such as cancers derived from colon, pancreas, brain, head and neck.

The mechanism of action of the anti-CD44 cannot be completely deduced from these data.

However analysis of protein extracts show that the MAP kinase pathway is probably involved in the anti-proliferative effect.

Moreover some cytokines with antitumor activity such as Oncostatin M are strongly overexpressed in treated tumors. Whereas this is a direct or indirect effect is not clear, but Oncostatin M has been shown to induce strong cytostasis in breast cancer cell lines (27).

Other mechanisms of action are possible, such as antibody-dependent cell cytotoxicity (ADCC) or modification of the CD44 interaction with the acid hyaluronic,. CD44 is a transmembrane protein which interacts with numerous proteins in the cytoplasm and in the extracellular space (26). The CD44 ligand is the hyaluronic acid (HA), a major component of the extracellular compartment (28). HA is responsible for the hydrophilic cell environment, facilitating the circulation and cellular access of cytokines and hydrosoluble growth factors. Increase of expression of CD44 induces an increase of HA, It can be postulated that survival of T-IC is in relation with access to growth factor, and circulation of effectors as angiogenic growth factor to attract efficiently endothelial cells necessary to implantation and growth. The anti-CD44 could act in removing HA from its receptor, reducing circulation of growth factors.

Another mechanism of action of the anti-CD44 Ab would be immunologic. Fixation of the Ab to the cancer cells could trigger an antibody-dependent cell cytotoxicity (ADCC) (29). This could be demonstrated by the loss of antitumoral effect using Fab or Fab'2 fragments of the P245 mAb. Our data showing the modifications of human cytokines in the tumors indicate that anti-CD44 alters signalisation pathways, either by a mechanism of deprivation or by induction of a new pathway (26). Apoptosis could be one of them.

This study illustrates an innovative concept of therapy, based on the inhibition of TIC which survive to drug-induced toxicity, and as shown by others to radiotherapy. It is possible to test this concept in any solid tumor types which show CD44 positive T-IC, as already known for colon, prostate, pancreas, head and neck and brain cancers. It is further conceivable that T-IC are present in other types of solid tumors, such as lung cancers, hepatomas, melanomas, lymphomas, endometrious, ovary, kidney, testis cancers, for which the phenotype is not yet known. According to the present study, such mAb therapy would be particularly useful in sequential combination of standard therapy, in any clinical situation in which a strong response is expected from standard therapy, and in which, nevertheless, relapses are frequent. It could also be postulated that such antibody therapy could reduce circulating T-IC nesting and proliferation and thus hinder metastasis.

In conclusion, the inventors demonstrate here that some tumor cells of human breast cancer xenografts express CD44, isolated or in grouped around microvessels under hypoxia conditions. They observed that an in vivo treatment with P245 mAb, an antibody directed to the CD44 transmembrane protein reduces the growth of human breast cancer xenografts, inducing the transcription of cytokines involved in cell proliferation inhibition. This antitumoral effect could be CD44-epitope dependent since the anti-Hermes 3 mAb, which recognizes a different epitope, had no effect. Finally, it is showed that in vivo treatment with P245 mAb prevented tumor relapse after chemotherapy.

### REFERENCES

1. Al-Hajj M, Wicha MS, Benito-Hernandez A, Morrison SJ, Clarke MF. Prospective identification of tumorigenic breast cancer cells. Proc Natl Acad Sci U S A. 2003 100:3983-8.
2. O'Brien CA, Pollett A, Gallinger S, Dick JE. A human colon cancer cell capable of initiating tumour growth in immunodeficient mice. Nature. 2007 Jan 4;445(7123):106-10.
3. Ponti D, Costa A, Zaffaroni N, Pratesi G, Petrangolini G, Coradini D, Pilotti S, Pierotti MA, Daidone MG. Isolation and in vitro propagation of tumorigenic breast cancer cells with stem/progenitor cell properties. Cancer Res. 2005 65:5506-11.
4. Ricci-Vitiani L, Lombardi DG, Pilozzi E, Biffoni M, Todaro M, Peschle C, De Maria R. Identification and expansion of human colon-cancer-initiating cells. Nature. 2007 Jan 4;445(7123):111-5.
5. Singh SK, Hawkins C, Clarke ID, Squire JA, Bayani J, Hide T, Henkelman RM, Cusimano MD, Dirks PB. Identification of human brain tumour initiating cells. Nature. 2004 Nov 18;432(7015):396-401.
6. Bao S, Wu Q, McLendon RE, Hao Y, Shi Q, Hjelmeland AB, Dewhirst MW, Bigner DD, Rich JN. Glioma stem cells promote radioresistance by preferential activation of the DNA damage response. Nature. 2006 Dec 7;444(7120):756-60. Epub 2006 Oct 18.
7. Lapidot T, Sirard C, Vormoor J, Murdoch B, Hoang T, Caceres-Cortes J, Minden M, Paterson B, Caligiuri MA, Dick JE. A cell initiating human acute myeloid leukaemia after transplantation into SCID mice. Nature. 1994 Feb 17;367(6464):645-8
8. Charrad RS, Li Y, Delpech B, Balitrand N, Clay D, Jasmin C, Chomienne C, Smadja-Joffe F. Ligation of the CD44 adhesion molecule reverses blockage of differentiation in human acute myeloid leukemia. Nat Med. 1999 Jun;5(6):669-76.
9. Patrawala L, Calhoun T, Schneider-Broussard R, Li H, Bhatia B, Tang S, Reilly JG, Chandra D, Zhou J, Claypool K, Coghlan L, Tang DG. Highly purified CD44+ prostate cancer cells from xenograft human tumors are enriched in tumorigenic and metastatic progenitor cells. Oncogene. 2006 25:1696-708.
10. Prince ME, Sivanandan R, Kaczorowski A, Wolf GT, Kaplan MJ, Dalerba P, Weissman IL, Clarke MF, Ailles LE. Identification of a subpopulation of cells with cancer stem cell properties in head and neck squamous cell carcinoma. Proc Natl Acad Sci U S A. 2007 Jan 8.
11. Balic M, Lin H, Young L, Hawes D, Giuliano A, McNamara G, Datar RH, Cote RJ. Most early disseminated cancer cells detected in bone marrow of breast cancer patients have a putative breast cancer stem cell phenotype. Clin Cancer Res. 2006 12:5615-21.
12. WO 03/050502
13. WO 2006/138275
14. Sheridan C, Kishimoto H, Fuchs RK, Mehrotra S, Bhat-Nakshatri P, Turner CH, Goulet R Jr, Badve S, Nakshatri H. CD44+/CD24- breast cancer cells exhibit enhanced invasive properties: an early step necessary for metastasis. Breast Cancer Res. 2006 Oct 24;8(5):
15. Verel I, Heider KH, Siegmund M, Ostermann E, Patzelt E, Sproll M, Snow GB, Adolf GR, van Dongen GA. Tumor targeting properties of monoclonal antibodies with different affinity for target antigen CD44V6 in nude mice bearing head-and-neck cancer xenografts. Int J Cancer. 2002 May 20;99(3):396-402
16. Charrad RS, Gadhoum Z, Qi J, Glachant A, Allouche M, Jasmin C, Chomienne C, Smadja-Joffe F. Effects of anti-CD44 monoclonal antibodies on differentiation and apoptosis of human myeloid leukemia cell lines. Blood. 2002 99:290-9.
17. Gadhoum Z, Leibovitch MP, Qi J, Dumenil D, Durand L, Leibovitch S, Smadja-Joffe F. CD44: a new means to inhibit acute myeloid leukemia cell proliferation via p27Kip1. Blood. 2004 Feb 1;103(3):1059-68.
18. Jin L, Hope KJ, Zhai Q, Smadja-Joffe F, Dick JE. Targeting of CD44 eradicates human acute myeloid leukemic stem cells. Nat Med. 2006 Oct;12(10):1167-74.
19. EP1532984
20. WO 2005/049082
21. Van Hal NL et al. Monoclonal antibody U36, a suitable candidate for clinical immunotherapy of squamous-cell carcinoma, recognizes a CD44 isoform. Int J Cancer. 1996 Nov 15;68(4):520-7.
22. Phillips TM, McBride WH, Pajonk F. The response of CD24(-/low)/CD44+ breast cancer-initiating cells to radiation. J Natl Cancer Inst. 2006 98:1777-85.
23. Abraham BK, Fritz P, McClellan M, Hauptvogel P, Athelogou M, Brauch H. Prevalence of CD44+/CD24-/low cells in breast cancer may not be associated with clinical outcome but may favor distant metastasis. Clin Cancer Res. 2005 11:1154-9
24. Draffin JE, McFarlane S, Hill A, Johnston PG, Waugh DJ. CD44 potentiates the adherence of metastatic prostate and breast cancer cells to bone marrow endothelial cells. Cancer Res. 2004 64:5702-11
25. Liu R, Wang X, Chen GY, Dalerba P, Gurney A, Hoey T, Sherlock G, Lewicki J, Shedden K, Clarke MF. The prognostic role of a gene signature from tumorigenic breast-cancer cells. N Engl J Med. 2007 56:217-26.
26. Ponta H, Sherman L, Herrlich PA. CD44: from adhesion molecules to signalling regulators. Nat Rev Mol Cell Biol. 2003 Jan;4(1):33-45.
27. Underhill-Day N, Heath JK. Oncostatin M (OSM) Cytostasis of Breast Tumor Cells: Characterization of an OSM Receptor B-Specific Kernel. Cancer Res 2006 66: 10891-901.
28. Gotte M, Yip GW. Heparanase, hyaluronan, and CD44 in cancers: a breast carcinoma perspective. Cancer Res. 2006 66:10233-7
29. Siberil S, Dutertre CA, Fridman WH, Teillaud JL. FcgammaR: The key to optimize therapeutic antibodies? Crit Rev Oncol Hematol. 2007 Apr;62(1):26-33.

## Claims

1. Use of an anti-CD44 antibody or an antigen binding fragment thereof for the manufacture of a drug for treating a solid tumor in a patient, in combination with another anti-cancer treatment.

2. Use of an anti-CD44 antibody or an antigen binding fragment thereof for the manufacture of a drug for preventing solid tumor relapse in a patient, in combination with another anti-cancer treatment.

3. Use of an anti-CD44 antibody or an antigen binding fragment thereof for the manufacture of a drug for preventing solid tumor metastasis in a patient, in combination with another anti-cancer treatment.

4. Use according to anyone of claims 1-3, wherein said patient is simultaneously treated or has been previously treated by said other anti-cancer treatment.

5. Use according to claim 4, wherein said patient has been previously treated by said other anti-cancer treatment.

6. Use according to claim 4, wherein said patient is simultaneously treated by said other anti-cancer treatment.

7. Use according to anyone of claims 1-6, wherein said other anti-cancer treatment is selected from the group consisting of chemotherapy, surgery, radiotherapy, hormonotherapy, and/or immunotherapy.

8. Use according to anyone of claims 1-7, wherein said other anti-cancer treatment is chemotherapy using at least one chemotherapeutic agent selected from the group consisting of topoisomerase inhibitors, anthracyclines, taxanes, anti-tubulines, anti-metabolite, and platinium salts.

9. Use according to anyone of claims 1-8, wherein said anti-CD44 antibody or antigen binding fragment thereof is a monoclonal antibody or an antigen binding fragment thereof.

10. Use according to anyone of claims 1-9, wherein said anti-CD44 antibody or antigen binding fragment thereof is a chimeric or humanized antibody or an antigen binding fragment thereof.

11. Use according to anyone of claims 1-10, wherein said antigen binding fragment thereof is selected from the group consisting of a Fab fragment, a (Fab')2 fragment, a single chain antibody such as a ScFv fragment, a disulfide-linked Fv, and a fragment containing either a VL or VH domain or even a complementary determining region (CDR).

12. Use according to anyone of claims 1-11, wherein said anti-CD44 antibody or antigen binding fragment thereof is specifically directed to an epitope located in SEQ ID NO:1.

13. Use according to claim 12, wherein said anti-CD44 antibody or antigen binding fragment thereof has a heavy chain comprising CDR1, CDR2, and CDR3 regions represented by GYAFSRYW (SEQ ID NO:4), IYPGDGDT (SEQ ID NO:5), and ARRRWSDYFGMDY (SEQ ID NO:6) respectively, and a light chain comprising CDR1, CDR2, and CDR3 regions represented by QSLLHSNGKTY (SEQ ID NO:7), LVS, and LQATHFPLT (SEQ ID NO:8) respectively.

14. Use according to claim 13, wherein said anti-CD44 antibody is selected from the group consisting of the P245 murine antibody which has a heavy chain represented by SEQ ID NO:2 and a light chain represented by SEQ ID NO:3; an IgG1 chimeric P245 antibody which has a heavy chain represented by SEQ ID NO:9 and a light chain represented by SEQ ID NO:10; or an IgG4 chimeric P245 antibody which has a heavy chain represented by SEQ ID NO:11 and a light chain represented by SEQ ID NO:10.

15. Use according to anyone of claims 1-14, wherein said solid tumor is selected from the group consisting of breast cancer, prostate cancer, colon cancer, head and neck cancer, ovary cancer, melanoma, small cell lung cancer, testis cancer, kidney cancer, pancreatic cancer, and osteosarcoma.

16. Use according to anyone of claims 1-15, wherein said anti-CD44 antibody is administered at a dose of less than 150 mg/kg/j.

17. Use according to anyone of claims 1-16, wherein said anti-CD44 antibody is continuously administered to said patient.

18. Use according to anyone of claims 1-17, wherein said anti-CD44 antibody is further administered in combination with an anti-angiogenic agent.

19. Use according to claim 18, wherein said anti-angiogenic agent is selected from the group consisting of an anti-VEGF antibody, an anti-VEGF receptor antibody, thalidomide, and SU 11657 (Sutent).


SEQUENCE LISTING

<110> Matbiopharma
      Institut National de la Santé et de la Recherche Médicale
      (INSERM)
      Centre National de la Recherche Scientifique (CNRS)
      Institut Curie

<120> Combination of a conventional anti-cancer treatment with
      anti-CD44 antibody administration for treating solid tumors

<130> 67064/D25687

<160> 11

<170> PatentIn version 3.3


<210> 1
<211> 90
<212> PRT
<213> artificial sequence

<220>
<223> HA to CD44 bonding region

<400> 1

Leu Cys Leu Val Pro Leu Ser Leu Ala Gln Ile Asp Leu Met Ile Thr
1               5                   10                  15
Cys Arg Phe Ala Gly Val Phe His Val Glu Lys Asn Gly Arg Tyr Ser
            20                  25                  30
Ile Ser Arg Thr Glu Ala Ala Asp Leu Cys Lys Ala Phe Asn Ser Thr
        35                  40                  45
Leu Pro Thr Met Ala Gln Met Glu Lys Ala Leu Ser Ile Gly Phe Glu
    50                  55                  60
Thr Cys Arg Tyr Gly Phe Ile Glu Gly His Val Val Ile Pro Arg Ile
65                  70                  75                  80
His Pro Asn Ser Ile Cys Ala Ala Asn Asn
                85                  90


<210> 2
<211> 444
<212> PRT
<213> artificial sequence

<220>
<223> murine P245 antibody heavy chain

<400> 2

Gln Val Gln Leu Gln Gln Ser Gly Asp Glu Leu Val Arg Pro Gly Ser
1               5                   10                  15
Ser Val Lys Ile Ser Cys Lys Ala Ser Gly Tyr Ala Phe Ser Arg Tyr
            20                  25                  30
Trp Met Asn Trp Val Lys Gln Arg Pro Gly Gln Gly Leu Glu Trp Ile
        35                  40                  45
Gly Gln Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80
Met Gln Leu Asn Ser Leu Thr Ser Glu Asp Ser Ala Val Tyr Phe Cys
                85                  90                  95
Ala Arg Arg Arg Trp Ser Asp Tyr Phe Gly Met Asp Tyr Trp Gly Gln
            100                 105                 110
Gly Thr Ser Val Thr Val Ser Ser Ala Lys Thr Thr Pro Pro Ser Val
        115                 120                 125
Tyr Pro Leu Ala Pro Gly Ser Ala Ala Gln Thr Asn Ser Met Val Thr
    130                 135                 140
Leu Gly Cys Leu Val Lys Gly Tyr Phe Pro Glu Pro Val Thr Val Thr
145                 150                 155                 160
Trp Asn Ser Gly Ser Leu Ser Ser Gly Val His Thr Phe Pro Ala Val
                165                 170                 175
Leu Glu Ser Asp Leu Tyr Thr Leu Ser Ser Ser Val Thr Val Pro Ser
            180                 185                 190
Ser Pro Arg Pro Ser Glu Thr Val Thr Cys Asn Val Ala His Pro Ala
        195                 200                 205
Ser Ser Thr Lys Val Asp Lys Lys Ile Val Pro Arg Asp Cys Gly Cys
    210                 215                 220
Lys Pro Cys Ile Cys Thr Val Pro Glu Val Ser Ser Val Phe Ile Phe
225                 230                 235                 240
Pro Pro Lys Pro Lys Asp Val Leu Thr Ile Thr Leu Thr Pro Lys Val
                245                 250                 255
Thr Cys Val Val Val Asp Ile Ser Lys Asp Asp Pro Glu Val Gln Phe
            260                 265                 270
Ser Trp Phe Val Asp Asp Val Glu Val His Thr Ala Gln Thr Gln Pro
        275                 280                 285
Arg Glu Glu Gln Phe Asn Ser Thr Phe Arg Ser Val Ser Glu Leu Pro
    290                 295                 300
Ile Met His Gln Asp Trp Leu Asn Gly Lys Glu Phe Lys Cys Arg Val
305                 310                 315                 320
Asn Ser Ala Ala Phe Pro Ala Pro Ile Glu Lys Thr Ile Ser Lys Thr
                325                 330                 335
Lys Gly Arg Pro Lys Ala Pro Gln Val Tyr Thr Ile Pro Pro Pro Lys
            340                 345                 350
Glu Gln Met Ala Lys Asp Lys Val Ser Leu Thr Cys Met Ile Thr Asp
        355                 360                 365
Phe Phe Pro Glu Asp Ile Thr Val Glu Trp Gln Trp Asn Gly Gln Pro
    370                 375                 380
Ala Glu Asn Tyr Lys Asn Thr Gln Pro Ile Met Asn Thr Asn Gly Ser
385                 390                 395                 400
Tyr Phe Val Tyr Ser Lys Leu Asn Val Gln Lys Ser Asn Trp Glu Ala
                405                 410                 415
Gly Asn Thr Phe Thr Cys Ser Val Leu His Glu Gly Leu His Asn His
            420                 425                 430
His Thr Glu Lys Ser Leu Ser His Ser Pro Gly Lys
        435                 440

<210> 3
<211> 219
<212> PRT
<213> artificial sequence

<220>
<223> murine P245 antibody light chain

<400> 3

Asp Val Val Met Thr Gln Thr Pro Leu Thr Leu Ser Val Thr Ile Gly

1               5                   10                  15
Gln Pro Ala Ser Ile Ser Cys Lys Ser Ser Gln Ser Leu Leu His Ser
            20                  25                  30
Asn Gly Lys Thr Tyr Leu Asn Trp Leu Leu Gln Arg Pro Gly Gln Ser
        35                  40                  45
Pro Lys Leu Leu Ile Tyr Leu Val Ser Lys Leu Glu Ser Gly Val Pro
    50                  55                  60
Asp Arg Phe Ser Gly Ser Gly Ser Gly Thr Glu Phe Thr Leu Lys Ile
65                  70                  75                  80
Ser Arg Val Glu Ala Glu Asp Ser Gly Val Tyr Tyr Cys Leu Gln Ala
                85                  90                  95
Thr His Phe Pro Leu Thr Phe Gly Ala Gly Thr Lys Leu Glu Leu Lys
            100                 105                 110
Arg Ala Asp Ala Ala Pro Thr Val Ser Ile Phe Pro Pro Ser Ser Glu
        115                 120                 125
Gln Leu Thr Ser Gly Gly Ala Ser Val Val Cys Phe Leu Asn Asn Phe
    130                 135                 140
Tyr Pro Lys Asp Ile Asn Val Lys Trp Lys Ile Asp Gly Ser Glu Arg
145                 150                 155                 160
Gln Asn Gly Val Leu Asn Ser Trp Thr Asp Gln Asp Ser Lys Asp Ser
                165                 170                 175
Thr Tyr Ser Met Ser Ser Thr Leu Thr Leu Thr Lys Asp Glu Tyr Glu
            180                 185                 190
Arg His Asn Ser Tyr Thr Cys Glu Ala Thr His Lys Thr Ser Thr Ser
        195                 200                 205
Pro Ile Val Lys Ser Phe Asn Arg Asn Glu Cys
    210                 215


<210> 4
<211> 8
<212> PRT
<213> artificial sequence

<220>
<223> murine P245 antibody heavy chain CDR1 region

<400> 4

Gly Tyr Ala Phe Ser Arg Tyr Trp
1               5


<210> 5
<211> 8
<212> PRT
<213> artificial sequence

<220>
<223> murine P245 antibody heavy chain CDR2 region

<400> 5

Ile Tyr Pro Gly Asp Gly Asp Thr
1               5


<210> 6
<211> 13
<212> PRT
<213> artificial sequence

<220>
<223> murine P245 antibody heavy chain CDR3 region

<400> 6

Ala Arg Arg Arg Trp Ser Asp Tyr Phe Gly Met Asp Tyr
1               5                   10


<210> 7
<211> 11
<212> PRT
<213> artificial sequence

<220>
<223> murine P245 antibody light chain CDR1 region

<400> 7

Gln Ser Leu Leu His Ser Asn Gly Lys Thr Tyr
1               5                   10


<210> 8
<211> 9
<212> PRT
<213> artificial sequence

<220>
<223> murine P245 antibody light chain CDR3 region

<400> 8

Leu Gln Ala Thr His Phe Pro Leu Thr
1               5


<210> 9
<211> 450
<212> PRT
<213> artificial sequence

<220>
<223> chimeric P245 antibody heavy IgG1 chain

<400> 9

Gln Val Gln Leu Gln Gln Ser Gly Asp Glu Leu Val Arg Pro Gly Ser
1               5                   10                  15
Ser Val Lys Ile Ser Cys Lys Ala Ser Gly Tyr Ala Phe Ser Arg Tyr
            20                  25                  30
Trp Met Asn Trp Val Lys Gln Arg Pro Gly Gln Gly Leu Glu Trp Ile
        35                  40                  45
Gly Gln Ile Tyr Pro Gly Asp Gly Asp Thr Asn Tyr Asn Gly Lys Phe
    50                  55                  60
Lys Gly Lys Ala Thr Leu Thr Ala Asp Lys Ser Ser Ser Thr Ala Tyr
65                  70                  75                  80
Met Gln Leu Asn Ser Leu Thr Ser Glu Asp Ser Ala Val Tyr Phe Cys
                85                  90                  95
Ala Arg Arg Arg Trp Ser Asp Tyr Phe Gly Met Asp Tyr Trp Gly Gln

                100                 105                 110
Gly Thr Ser Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450

<210> 10
<211> 219
<212> PRT
<213> artificial sequence

<220>
<223> chimeric P245 antibody light kappa chain

<400> 10

Asp Val Val Met Thr Gln Thr Pro Leu Thr Leu Ser Val Thr Ile Gly
1               5                   10                  15
Gln Pro Ala Ser Ile Ser Cys Lys Ser Ser Gln Ser Leu Leu His Ser
            20                  25                  30

Asn Gly Lys Thr Tyr Leu Asn Trp Leu Leu Gln Arg Pro Gly Gln Ser
        35                  40                  45
Pro Lys Leu Leu Ile Tyr Leu Val Ser Lys Leu Glu Ser Gly Val Pro
    50                  55                  60
Asp Arg Phe Ser Gly Ser Gly Ser Gly Thr Glu Phe Thr Leu Lys Ile
65                  70                  75                  80
Ser Arg Val Glu Ala Glu Asp Ser Gly Val Tyr Tyr Cys Leu Gln Ala
                85                  90                  95
Thr His Phe Pro Leu Thr Phe Gly Ala Gly Thr Lys Leu Glu Leu Lys
            100                 105                 110
Arg Thr Val Ala Ala Pro Ser Val Phe Ile Phe Pro Pro Ser Asp Glu
        115                 120                 125
Gln Leu Lys Ser Gly Thr Ala Ser Val Val Cys Leu Leu Asn Asn Phe
    130                 135                 140
Tyr Pro Arg Glu Ala Lys Val Gln Trp Lys Val Asp Asn Ala Leu Gln
145                 150                 155                 160
Ser Gly Asn Ser Gln Glu Ser Val Thr Glu Gln Asp Ser Lys Asp Ser
                165                 170                 175
Thr Tyr Ser Leu Ser Ser Thr Leu Thr Leu Ser Lys Ala Asp Tyr Glu
            180                 185                 190
Lys His Lys Val Tyr Ala Cys Glu Val Thr His Gln Gly Leu Ser Ser
        195                 200                 205
Pro Val Thr Lys Ser Phe Asn Arg Gly Glu Cys
    210                 215


<210> 11
<211> 447
<212> PRT
<213> artificial sequence

<220>
<223> chimeric P245 antibody heavy IgG4 chain

<400> 11

Gln Val Gln Leu Gln Gln Ser Gly Asp Glu Leu Val Arg Pro Gly Ser
1               5                   10                  15
Ser Val Lys Ile Ser Cys Lys Ala Ser Gly Tyr Ala Phe Ser Arg Tyr
            20                  25                  30
Trp Met Asn Trp Val Lys Gln Arg Pro Gly Gln Gly Leu Glu Trp Ile
        35                  40                  45
Gly Gln Ile Tyr Pro Gly Asp Gly Asp Thr Asn Tyr Asn Gly Lys Phe
    50                  55                  60
Lys Gly Lys Ala Thr Leu Thr Ala Asp Lys Ser Ser Ser Thr Ala Tyr
65                  70                  75                  80
Met Gln Leu Asn Ser Leu Thr Ser Glu Asp Ser Ala Val Tyr Phe Cys
                85                  90                  95
Ala Arg Arg Arg Trp Ser Asp Tyr Phe Gly Met Asp Tyr Trp Gly Gln
            100                 105                 110
Gly Thr Ser Val Thr Val Ser Ser Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys

195                 200                 205
Pro Ser Asn Thr Lys Val Asp Lys Arg Val Glu Ser Lys Tyr Gly Pro
    210                 215                 220
Pro Cys Pro Ser Cys Pro Ala Pro Glu Phe Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Lys
        435                 440                 445